# EUROPEAN PATENT APPLICATION

(11) **EP 4 516 302 A1**
(43) Date of publication of application: **05.03.2025**
(21) Application number: 23195244.1
(22) Date of filing: 04.09.2023
(51) Int. Cl.: A61K 31/465, A61P 25/30, A24B 15/16, A61K 9/00

(54) **(S)-2-METHYL-5-(1-METHYL-2-PYRROLIDINYL)PYRIDINE, COMPOSITION AND ITS USE FOR TREATING NICOTINE DEPENDENCE**

(71) Applicant: Shesternenko, Dmitrii, 40-534 Katowice (PL)
(72) Inventor: Shesternenko, Dmitrii, 40-534 Katowice (PL)
(74) Representative: JD&P Patent Attorneys Joanna Dargiewicz & Partners

(57) **Abstract**

An subject of the present invention is (S)-2-Methyl-5-(1-methyl-2-pyrrolidinyl)pyridine for use in in a method for the treatment of nicotine dependence in an amount of 0.1-5% by weight, a pharmaceutical composition comprising (S)-2-Methyl-5-(1-methyl-2-pyrrolidinyl)pyridine for use in a method for the treatment of nicotine addiction in an amount of 0.1-5% by weight per composition of oral, inhalation and dermal products for humans.

It is also an subject of the present invention to use a composition comprising (S)-2-Methyl-5-(1-methyl-2-pyrrolidinyl)pyridine in an amount of 0.1-5% by weight as a substitute for a nicotine composition in food products, inhalation and dermal products for humans, the composition being in liquid or solid form and selected from such as e-cigarettes, e-cigarette liquids, chewing tobaccos, oral pouches, chewing gums, candies, lollipops, strips, dragees, jelly beans, lozenges, tablets, snuffs, shisha-type smoking mixtures, toothpastes, inhalation mixtures, inhalers or skin patches.

## Description

The present invention relates to (S)-2-Methyl-5-(1-methyl-2-pyrrolidinyl)pyridine and compositions comprising it for the treatment of nicotine dependence, the use of (S)-2-Methyl-5-(1-methyl-2-pyrrolidinyl)pyridine and compositions comprising it as a substitute for nicotine compositions in products, especially oral, inhalation and dermal products for humans, and the composition of (S)-2-Methyl-5-(1-methyl-2-pyrrolidinyl)pyridine.

### BACKGROUND OF THE INVENTION

Discontinuation of nicotine use is extremely difficult for many smokers, and many who seek help to quit in formal programs return to smoking within a year. Although nicotine withdrawal causes symptoms that are most intense 48-72 hours after cessation, such as increased anxiety, hostility and depression, in the long term the former smoker is at risk for weight gain and a desire to return to smoking.

Currently, the industry can categorize nicotine products that comprise nicotine, nicotine derivatives, nicotine salts or acid-nicotine mixtures. The content of the aforementioned substances in products is considered toxic to the body. The most popular concentrations are 2% (legal in the EU) and 5% (illegal in the EU, but available) for nicotine, available in products such as gums, transdermal patches, nasal and inhalation sprays, oral strip types, lollipops, jellies, lozenges, tablets, pastes and pouches put into the mouth.

Nicotine, 3-[(2S)-1-methyl-2-pyrrolidinyl]pyridine, of molecular formula C₁₀H₁₄N₂, CAS No. 54-11-5; is an organic chemical compound from the pyridine alkaloid group. Nicotine is a potent and agonist of several subpopulations of the cholinergic nicotinic receptors of the cholinergic nervous system (Henningfield, Keenan and Clarke, 1996; Vidal, 1996; Paterson and Nordberg, 2000). Pure nicotine is a clear oily liquid with a distinctive, potent odour that turns brown in air. Nicotine is an amine consisting of two heterocyclic rings, pyridine and pyrrolidine, with its carbon atom at position 2 being the chiral centre. Accordingly, there are two optical isomers (enantiomers) of nicotine which rotate polarised light in opposite directions. Furthermore, the majority of physical and chemical characteristics for both enantiomers are essentially equivalent. In the case of nicotine, the natural product is dominated by the left-handed (S) enantiomer, conventionally referred to as L- or (-)-nicotine. The content of the right-handed (R)-enantiomer of nicotine (also referred to as D- or (+)-nicotine) typically does not exceed a few percent (and its biological activity is approximately 8 times lower):

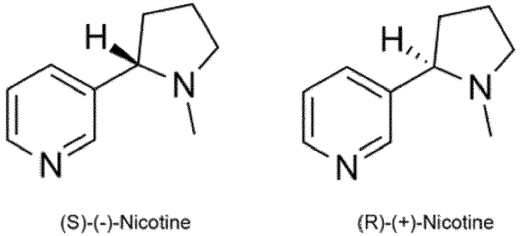

### Chemical structure of the enantiomers of nicotine: (S)-nicotine and (R)-nicotine

Nicotine typically accounts for about 5% of the weight of tobacco. Nicotine acts on nicotinic cholinergic receptors, affects most organ systems in the body and is highly addictive. It has been found to play a role in the development of cardiovascular disease and fertility disorders, and is a major precursor of the highly carcinogenic tobacco-specific N-nitrosamines. The lethal dose for humans is approximately 50-100 mg (https://chemia.ug.edu.pl/sites/default/files/_nodes/strona-chemia/14098/files/analiza_zawartosci_nikotyny_w_lisciach_tytoniu_oraz_wyrobach_tytoniowyc h_z_wykorzystaniem_techniki_elektroforezy_kapilarnej_ce_i_ultra-wysokosprawnej_chromatografii_cieczowej_z_detekcja_spektrometrii_mas_lc-ms.pdf).

The optically active analogue of nicotine, (S)-2-Methyl-5-(1-methyl-2-pyrrolidinyl)pyridine, (also known as (S)-6-methylnicotine), is also known from the state of the art, with CAS 13270-56-9, of the molecular formula C₁₁H₁₆N₂, as an orange-coloured oil with the following structural formula:

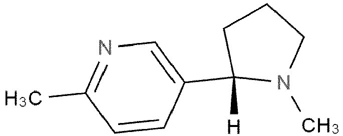

In a publication Ramunno, A. et al.: Bioorg, Med. Chem. Lett., 15, 3237, 2005, it was revealed that (S)-2-Methyl-5-(1-methyl-2-pyrrolidinyl)pyridine displays antinociceptive effects when interacting with the cholinergic receptor ligand.

A synthetic route to obtain 2-methyl-5-(1-methyl-2-pyrrolidinyl)pyridine is presented in J. Org. Chem. 1983,48, 4899-4904, using a reaction with a methyllithium, a strong nucleophile, in one of the organic solvents (e.g. toluene), which leads to the isolation of (S)-2-Methyl-5-(1-methyl-2-pyrrolidinyl)pyridine with small amounts of 4-methylnicotine.

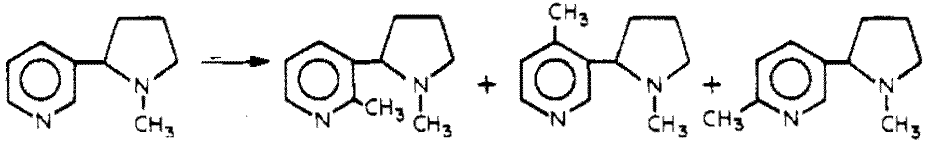

Another synthetic method to obtain (S)-2-Methyl-5-(1-methyl-2-pyrrolidinyl)pyridine is presented in patent description US4321387.

Recent reports from the available literature, report that studies have been completed to assess the cytotoxicity of nicotine and its analogue (S)-2-Methyl-5-(1-methyl-2-pyrrolidinyl)pyridine on human bronchial epithelial cells (BEAS-2B cells) in vitro. Transcriptome sequencing was also performed to systematically assess the effects of nicotine and (S)-2-Methyl-5-(1-methyl-2-pyrrolidinyl)pyridine on BEAS-2B cells. The cytotoxicity assay showed that BEAS-2B cells were more sensitive to (S)-2-Methyl-5-(1-methyl-2-pyrrolidinyl)pyridine than to nicotine. Transcriptome sequencing revealed 1208 cancer-related proteins (CRPs) with differential expression in the (S)-2-Methyl-5-(1-methyl-2-pyrrolidinyl)pyridine groups compared with CRP proteins in the control group. Furthermore, (S)-2-Methyl-5-(1-methyl-2-pyrrolidinyl)pyridine had a greater negative effect on CRP expression than nicotine. Bioinformatics analysis revealed that genes and proteins differentially expressed in the (S)-2-Methyl-5-(1-methyl-2-pyrrolidinyl)pyridine group were significantly enriched in cancer-related pathways, in contrast to those in the nicotine group. Further validations of some lung cancer-associated proteins, such as NF-κB p65, EGFR and MET, were conducted by immunoblotting and real-time PCR, which revealed that (S)-2-Methyl-5-(1-methyl-2-pyrrolidinyl)pyridine may have a greater negative effect on tumour development and metastasis than nicotine. In summary, the latest findings suggest that products comprising (S)-2-Methyl-5-(1-methyl-2-pyrrolidinyl)pyridine may offer some advantages over conventional nicotine-containing products (QI, Huaiyuan, et al. Comparative analyses of transcriptome sequencing and carcinogenic exposure toxicity of nicotine and 6-methyl nicotine in human bronchial epithelial cells. Toxicology in Vitro, 2023, 105661).

(S)-2-Methyl-5-(1-methyl-2-pyrrolidinyl)pyridine also had applications as a reactant in the synthesis of derivatives such as 6-(2-phenylethyl)nicotine, in order to find a new ligand for the nicotinic cholinergic receptor.

Due to the adverse health effects of smoking and the difficulty many smokers have in quitting completely, it would be beneficial to have an effective method of quitting smoking and, moreover, moving away from nicotine, which is highly addictive and toxic.

### SUMMARY OF THE INVENTION

Unexpectedly, it has been found that (S)-2-Methyl-5-(1-methyl-2-pyrrolidinyl)pyridine could effectively replace nicotine in existing products.

Therefore the subject of the present invention is (S)-2-Methyl-5-(1-methyl-2-pyrrolidinyl)pyridine, in an amount of 0.1-5% by weight of the composition, for use in a method for the treatment of nicotine dependence.

The subject of the invention is also a pharmaceutical composition comprising (S)-2-Methyl-5-(1-methyl-2-pyrrolidinyl)pyridine, in an amount of 0.1-5% by weight of the composition of oral, inhalation and dermal products for humans, for use in a method for the treatment of nicotine dependence. Preferably this composition is in the form of a liquid or a solid. Preferably, the composition is in the form of an oral, inhalation or dermal human product selected from such as e-cigarettes, e-cigarette liquids, chewing tobaccos, oral pouches, chewing gums, candies, lollipops, oral strips, dragees, jelly beans, lozenges, tablets, snuffs, shisha type smoking mixes, toothpastes, inhalation mixes, inhalers or skin patches. It is also preferred, that the said composition comprises excipients selected from such as at least one substance responsible for the flavour, 1,2-propanediol, N,2,3-trimethyl-2-isopropylbutamide, cellulose, sodium chloride, glycerol and the like. The above-mentioned composition may additionally comprise nicotine or its derivatives, in an amount of 0.01-5% by weight of the composition.

Another subject of the present invention is use of a composition comprising (S)-2-Methyl-5-(1-methyl-2-pyrrolidinyl)pyridine in an amount of 0.1-5 % by weight as a substitute for nicotine composition in food, inhalation and dermal products for humans. Preferably, then, the composition is in the form of a liquid or a solid. Also preferably, the said composition is in the form of an oral, inhalation or dermal human product selected from such as e-cigarettes, e-cigarette liquids, chewing tobaccos, oral pouches, chewing gums, candies, lollipops, oral strips, dragees, jelly beans, lozenges, tablets, snuffs, shisha type smoking mixes, toothpastes, inhalation mixes, inhalers or skin patches. Another preferred aspect of the invention is that the said composition comprises excipients selected from such as at least one substance responsible for the flavour, 1,2-propanediol, N,2,3-trimethyl-2-isopropylbutamide, cellulose, sodium chloride, glycerol and the like. Also preferably, the composition additionally comprises nicotine or its derivatives in an amount of 0.01-5% by weight of the composition.

Yet the invention also presents use of (S)-2-Methyl-5-(1-methyl-2-pyrrolidinyl)pyridine in an amount of 0.1-5% by weight, as a substitute for nicotine or its derivatives in food, inhalation and dermal products for humans.

Also the subject of the present invention is a composition comprising (S)-2-Methyl-5-(1-methyl-2-pyrrolidinyl)pyridine in an amount of 0.1-5% by weight and excipients selected from such as at least one substance responsible for the flavour, 1,2-propanediol, N,2,3-trimethyl-2-isopropylbutamide, cellulose, sodium chloride, glycerol and the like. Preferably, this composition is in the form of a liquid or a solid in products selected from such as e-cigarettes, e-cigarette liquids, chewing tobaccos, oral pouches, chewing gums, candies, lollipops, oral strips, dragees, jelly beans, lozenges, tablets, snuffs, shisha type smoking mixes, toothpastes, inhalation mixes, inhalers or skin patches. Also preferably, the said composition additionally comprises nicotine or its derivatives in an amount of 0.01-5% by weight.

### DETAILED DESCRIPTION OF THE INVENTION

The subject of the invention is use of (S)-2-Methyl-5-(1-methyl-2-pyrrolidinyl)pyridine in an amount of 0.1-5 % by weight of the composition as a substitute (total or partial) for nicotine and its derivatives in food, inhalation and dermal products for humans.

The use, in particular, may relate to the use of the compound (S)-2-Methyl-5-(1-methyl-2-pyrrolidinyl)pyridine in as e-cigarettes, e-cigarette liquids, chewing tobaccos, oral pouches, chewing gums, candies, lollipops, oral strips, dragees, jelly beans, lozenges, tablets, snuffs, shisha type smoking mixes, toothpastes, inhalation mixes, inhalers or skin patches.

The subject of the invention is presented in detail in the below-given embodiments. The following execution examples are for illustrative purposes only and are not intended to limit the scope of the invention in any manner.

### Example 1- compositions with (S)-2-Methyl-5-(1-methyl-2-pyrrolidinyl)pyridine for use as a substitute for nicotine in the form of e-cigarette liquid, form of the compositions: liquid

| **Composition 1: Strawberry Ice Cream** | | |
|---|---|---|
| **Component** | **CAS No.** | **Content [wt.%]** |
| 1,2-Propanediol | 57-55-6 | 44.2 |
| Glycerol | 56-81-5 | 39.4 |
| N,2,3-Trimethyl-2-isopropylbutamide | 51115-67-4 | 6.2 |
| Ethanol | 64-17-5 | 4.0 |
| Ethyl maltol | 4940-11-8 | 2.7 |
| (S)-2-Methyl-5-(1-methyl-2-pyrrolidinyl)pyridine | 13270-56-9 | 0.9 |
| Vanillin | 121-33-5 | 0.9 |
| gamma-Nonanolactone | 104-61-0 | 0.6 |
| Ethyl Vanillin | 121-32-4 | 0.3 |
| Ethyl butyrate | 105-54-4 | 0.2 |
| cis-3-Hexen-1-ol | 928-96-1 | 0.2 |
| Methyl cinnamate | 103-26-4 | 0.2 |
| Methyl dihydrojasmonate | 24851-98-7 | 0.2 |
| | | |

| **Composition 2: Cherry Ice** | | |
|---|---|---|
| Component | CAS No. | Content [wt.%] |
| 1,2-Propanediol | 57-55-6 | 54.3 |
| Glycerol | 56-81-5 | 36.6 |
| N,2,3-Trimethyl-2-isopropylbutamide | 51115-67-4 | 6.8 |
| (S)-2-Methyl-5-(1-methyl-2-pyrrolidinyl)pyridine | 13270-56-9 | 0.9 |
| Methyl dihydrojasmonate | 24851-98-7 | 0.4 |
| Vanillin | 121-33-5 | 0.4 |
| 2-Hydroxypropylacetate | 627-69-0 | 0.2 |
| 2,5-Dimethyl-4-hydroxy-3[2H]-furanone | 3658-77-3 | 0.1 |
| acetate, (E)-2-Hexen-1-ol | 2497-18-9 | 0.1 |
| Isoamyl acetate | 123-92-2 | 0.05 |
| | | |

| **Composition 3: Strawberry Ice** | | |
|---|---|---|
| **Component** | **CAS No.** | **Content [wt.%]** |
| 1,2-Propanediol | 57-55-6 | 52.7 |
| Glycerol | 56-81-5 | 38.8 |
| N,2,3-Trimethyl-2-isopropylbutamide | 51115-67-4 | 3.9 |
| (S)-2-Methyl-5-(1-methyl-2-pyrrolidinyl)pyridine | 13270-56-9 | 0.9 |
| Ethanol | 64-17-5 | 1.4 |
| Vanillin | 121-33-5 | 0.4 |
| Ethyl butyrate | 105-54-4 | 0.4 |
| Ethyl maltol | 4940-11-8 | 0.3 |
| Butanoic acid, 2-methyl-, ethyl ester | 7452-79-1 | 0.3 |
| γ-Decalactone | 706-14-9 | 0.3 |
| Hexanoic acid, ethyl ester | 123-66-0 | 0.2 |
| cis-3-Hexen-1-ol | 928-96-1 | 0.2 |
| Methyl cinnamate | 103-26-4 | 0.2 |
| Butyric acid | 107-92-6 | 0.1 |

| **Composition 4: Lychee Ice** | | |
|---|---|---|
| **Component** | **CAS No.** | **Content [wt.%]** |
| 1,2-Propanediol | 57-55-6 | 52.4 |
| Glycerol | 56-81-5 | 38.0 |
| N,2,3-Trimethyl-2-isopropylbutamide | 51115-67-4 | 7.8 |
| (S)-2-Methyl-5-(1-methyl-2-pyrrolidinyl)pyridine | 13270-56-9 | 0.9 |
| Benzyl alcohol | 100-51-6 | 0.4 |
| Diethyl succinate | 123-25-1 | 0.2 |
| Isoamyl acetate | 123-92-2 | 0.1 |
| Ethyl maltol | 4940-11-8 | 0.1 |
| cis-3-Hexen-1-ol | 928-96-1 | 0.1 |
| Eucalyptol | 470-82-6 | 0.1 |
| 2-Buten-1-ol, 3-methyl-, acetate | 1191-16-8 | 0.05 |
| δ-Decalactone | 705-86-2 | 0.02 |
| | | |

| **Composition 5: Mango Apple Pear** | | |
|---|---|---|
| **Component** | **CAS No.** | **Content [wt.%]** |
| 1,2-Propanediol | 57-55-6 | 48.2 |
| Glycerol | 56-81-5 | 39.5 |
| N,2,3-Trimethyl-2-isopropylbutamide | 51115-67-4 | 7.5 |
| (S)-2-Methyl-5-(1-methyl-2-pyrrolidinyl)pyridine | 13270-56-9 | 0.9 |
| Ethanol | 64-17-5 | 1.8 |
| Butyl acetate | 123-86-4 | 0.3 |
| Butanoic acid, 2-methyl-, ethyl ester | 7452-79-1 | 0.3 |
| (E)4-methyl-2-(pent-en-1-yl)-1,3-dioxolane | 94089-21-1 | 0.3 |
| cis-3-Hexen-1-ol | 928-96-1 | 0.2 |
| (Z)4-methyl-2-(pent-en-1-yl)-1,3-dioxolane | 94089-21-1 | 0.2 |
| Isoamyl acetate | 123-92-2 | 0.2 |
| Ethyl butyrate | 105-54-4 | 0.2 |
| 1,3-Dioxolane,4-methyl-2-pentyl | 1599-49-1 | 0.2 |
| Amyl butyrate | 540-18-1 | 0.2 |
| | | |

| **Composition 6: Berry Peach** | | |
|---|---|---|
| **Component** | **CAS No.** | **Content [wt.%]** |
| 1,2-Propanediol | 57-55-6 | 48.8 |
| Glycerol | 56-81-5 | 39.1 |
| N,2,3-Trimethyl-2-isopropylbutamide | 51115-67-4 | 6.5 |
| Ethanol | 64-17-5 | 2.6 |
| (S)-2-Methyl-5-(1-methyl-2-pyrrolidinyl)pyridine | 13270-56-9 | 0.9 |
| Benzyl alcohol | 100-51-6 | 0.6 |
| Isoamyl acetate | 123-92-2 | 0.4 |
| Ethyl butyrate | 105-54-4 | 0.2 |
| γ-Decalactone | 706-14-9 | 0.2 |
| cis-3-Hexen-1-ol | 928-96-1 | 0.2 |
| N-Ethyl-p-menthane-3-carboxamide | 39711-79-0 | 0.2 |
| Linalool | 78-70-6 | 0.1 |
| Isopentyl isopentanoater | 659-70-1 | 0.1 |
| 1-Butanol, 3-methyl- | 123-51-3 | 0.1 |
| | | |

| **Composition 7: Blueberry Bubble gum** | | |
|---|---|---|
| **Component** | **CAS No.** | **Content [wt.%]** |
| 1,2-Propanediol | 57-55-6 | 51.9 |
| Glycerol | 56-81-5 | 38.2 |
| N,2,3-Trimethyl-2-isopropylbutamide | 51115-67-4 | 6.0 |
| (S)-2-Methyl-5-(1-methyl-2-pyrrolidinyl)pyridine | 13270-56-9 | 0.9 |
| Isoamyl acetate | 123-92-2 | 1.0 |
| Ethyl butyrate | 105-54-4 | 0.6 |
| Ethanol | 64-17-5 | 0.4 |
| Isobutyl acetate | 110-19-0 | 0.3 |
| Cyclohexene,1-methyl-4-(1-methylethenyl)- | 5989-27-5 | 0.2 |
| Isoamyl butyraten | 106-27-4 | 0.2 |
| Linalool | 78-70-6 | 0.1 |
| 1-Butanol, 3-methyl- | 123-51-3 | 0.1 |
| cis-3-Hexen-1-ol | 928-96-1 | 0.1 |
| | | |

| **Composition 8a: Blueberry Pomegranate** | | |
|---|---|---|
| **Component** | **CAS No.** | **Content [wt.%]** |
| 1,2-Propanediol | 57-55-6 | 55.4 |
| Glycerol | 56-81-5 | 36.4 |
| N,2,3-Trimethyl-2-isopropylbutamide | 51115-67-4 | 5.9 |
| (S)-2-Methyl-5-(1-methyl-2-pyrrolidinyl)pyridine | 13270-56-9 | 0.9 |
| Isoamyl acetate | 123-92-2 | 0.4 |
| Ethyl butyrate | 105-54-4 | 0.3 |
| Vanillin | 121-33-5 | 0.2 |
| Isobutyl acetate | 110-19-0 | 0.2 |
| 1-Butanol, 3-methyl- | 123-51-3 | 0.1 |
| Linalool | 78-70-6 | 0.1 |
| γ-Decalactone | 706-14-9 | 0.1 |
| | | |

| **Composition 8b: Strawberry Guava** | | |
|---|---|---|
| **Component** | **CAS No.** | **Content [wt.%]** |
| 1,2-Propanediol | 57-55-6 | 47.0 |
| Glycerol | 56-81-5 | 43.6 |
| N,2,3-Trimethyl-2-isopropylbutamide | 51115-67-4 | 5.7 |
| (S)-2-Methyl-5-(1-methyl-2-pyrrolidinyl)pyridine | 13270-56-9 | 0.9 |
| Ethyl butyrate | 105-54-4 | 0.6 |
| Ethyl cinnamate | 103-36-6 | 0.5 |
| γ-Decalactone | 706-14-9 | 0.4 |
| cis-3-Hexen-1-ol | 928-96-1 | 0.3 |
| Butanoic acid, 2-methyl-, ethyl ester | 7452-79-1 | 0.3 |
| Hexanoic acid, ethyl ester | 123-66-0 | 0.2 |
| Methyl cinnamate | 103-26-4 | 0.2 |
| 2,5-Dimethyl-4-hydroxy-3[2H]-furanone | 3658-77-3 | 0.1 |
| 6-Methyl-5-hepten-2-one | 110-93-0 | 0.1 |
| Triacetin | 102-76-1 | 0.1 |
| | | |

| **Composition 9: Unicon shake** | | |
|---|---|---|
| **Component** | **CAS No.** | **Content [wt.%]** |
| Glycerol | 56-81-5 | 43.2 |
| 1,2-Propanediol | 57-55-6 | 40.4 |
| Isoamyl acetate | 123-92-2 | 6.1 |
| N,2,3-Trimethyl-2-isopropylbutamide | 51115-67-4 | 5.5 |
| (S)-2-Methyl-5-(1-methyl-2-pyrrolidinyl)pyridine | 13270-56-9 | 0.9 |
| Ethyl butyrate | 105-54-4 | 0.6 |
| Butyl acetate | 123-86-4 | 0.5 |
| cis-3-Hexen-1-ol | 928-96-1 | 0.5 |
| Butanoic acid, 2-methyl-, ethyl ester | 7452-79-1 | 0.4 |
| (E)4-methyl-2-(pent-en-1-yl)-1,3-dioxolane | 94089-21-1 | 0.4 |
| Isopentyl isopentanoater | 659-70-1 | 0.4 |
| (Z)4-methyl-2-(pent-en-1-yl)-1,3-dioxolane | 94089-21-1 | 0.3 |
| 1,3-Dioxolane,4-methyl-2-pentyl | 1599-49-1 | 0.3 |
| Isoamyl butyraten | 106-27-4 | 0.3 |

### Example 2 - compositions with (S)-2-Methyl-5-(1-methyl-2-pyrrolidinyl)pyridine for use in a nicotine pouch, form of the compositions: solid

| **Composition 10: Exotic Ice** | | |
|---|---|---|
| **Component** | **CAS No.** | **Content [wt.%]** |
| Cellulose (pure) | 9004-34-6 | 65 |
| Aqua | 7732-18-5 | 11 |
| 1,2-Propanediol | 57-55-6 | 2 |
| N,2,3-Trimethyl-2-isopropylbutamide | 51115-67-4 | 2 |
| Sucrose | 57-50-1 | 1 |
| Sodium chloride | 7647-14-5 | 1 |
| (S)-2-Methyl-5-(1-methyl-2-pyrrolidinyl)pyridine | 13270-56-9 | 1 |
| Potassium carbonate | 584-08-7 | 0.5 |
| Mentha arvensis, ext. | 90063-97-1 | 0.5 |
| Cyclohexene,1-methyl-4-(1-methylethenyl)- | 5989-27-5 | 0.36 |
| L-menthol | 2216-51-5 | 0.15 |
| trans-Menthone | 89-80-5 | 0.08 |
| Linalool | 78-70-6 | 0.04 |
| (E)-1-(2,6,6-trimethyl-1-cyclohexen-1-yl)-2-buten-1-one | 23726-91-2 | 0.04 |
| 7-methyl-3-methyleneocta-1,6-diene | 123-35-3 | 0.04 |
| Geranyl isobutyrate | 2345-26-8 | 0.04 |
| Lime (Citrus aurantifolia), ext. | 90063-52-8 | 0.04 |
| | | |

| **Composition 11: Freeze Ice** | | |
|---|---|---|
| **Component** | **CAS No.** | **Content [wt.%]** |
| Cellulose (pure) | 9004-34-6 | 66 |
| Aqua | 7732-18-5 | 13.15 |
| N,2,3-Trimethyl-2-isopropylbutamide | 51115-67-4 | 6 |
| L-menthol | 2216-51-5 | 4.5 |
| Sodium chloride | 7647-14-5 | 3 |
| 1,2-Propanediol | 57-55-6 | 3 |
| Plant oils / Spear mint oil | 8008-79-5 | 2 |
| (S)-2-Methyl-5-(1-methyl-2-pyrrolidinyl)pyridine | 13270-56-9 | 1 |
| L-menthan-3-one | 14073-97-3 | 0.5 |
| Potassium carbonate | 584-08-7 | 0.5 |
| Ammonium chloride | 12125-02-9 | 0.4 |
| Anise alcohol | 105-13-5 | 0.3 |
| Menthyl acetate | 16409-45-3 | 0.08 |
| Neomenthol | 491-01-0 | 0.08 |
| I-carvone | 6485-40-1 | 0.08 |
| Caryophyllene | 87-44-5 | 0.04 |
| (S)-p-mentha-1,8-diene | 5989-54-8 | 0.04 |
| | | |

| **Composition 12: Mango Ice** | | |
|---|---|---|
| **Component** | **CAS No.** | **Content [wt.%]** |
| Cellulose (pure) | 9004-34-6 | 68 |
| Aqua | 7732-18-5 | 14.5 |
| 1,2-Propanediol | 57-55-6 | 3 |
| N,2,3-Trimethyl-2-isopropylbutamide | 51115-67-4 | 2 |
| (S)-2-Methyl-5-(1-methyl-2-pyrrolidinyl)pyridine | 13270-56-9 | 1 |
| Sucrose | 57-50-1 | 1 |
| Sodium chloride | 7647-14-5 | 1 |
| Potassium carbonate | 584-08-7 | 0.5 |
| cis-3-Hexen-1-ol | 928-96-1 | 0.45 |
| Benzyl alcohol | 100-51-6 | 0.45 |
| Ethyl maltol | 4940-11-8 | 0.45 |
| 2,5-Dimethyl-4-hydroxy-3[2H]-furanone | 3658-77-3 | 0.45 |
| Hexanoic acid, ethyl ester | 123-66-0 | 0.45 |
| Isoamyl acetate | 123-92-2 | 0.45 |
| Citrus Grandis (Grapefruit) Peel Oil | 8016-20-4 | 0.23 |
| Allyl hexanoate | 123-68-2 | 0.23 |
| Methyl cinnamate | 103-26-4 | 0.043 |
| Cinnamaldehyde | 104-55-2 | 0.043 |
| Linalool | 78-70-6 | 0.043 |
| | | |

| **Composition 13: Rainbow Drops** | | |
|---|---|---|
| **Component** | **CAS No.** | **Content [wt.%]** |
| Cellulose (pure) | 9004-34-6 | 69 |
| Aqua | 7732-18-5 | 12 |
| Benzyl alcohol | 100-51-6 | 3 |
| 1,2-Propanediol | 57-55-6 | 2.5 |
| N,2,3-Trimethyl-2-isopropylbutamide | 51115-67-4 | 2 |
| 4-(4-hydroxyphenyl)butan-2-one | 5471-51-2 | 1.1 |
| (S)-2-Methyl-5-(1-methyl-2-pyrrolidinyl)pyridine | 13270-56-9 | 1 |
| Sucrose | 57-50-1 | 1 |
| Sodium chloride | 7647-14-5 | 1 |
| Potassium carbonate | 584-08-7 | 0.5 |
| Allyl 3-cyclohexylpropionate | 2705-87-5 | 0.28 |
| Ethyl maltol | 4940-11-8 | 0.28 |
| Hexanoic acid, ethyl ester | 123-66-0 | 0.28 |
| 2,5-Dimethyl-4-hydroxy-3[2H]-furanone | 3658-77-3 | 0.11 |
| Citral | 5392-40-5 | 0.11 |
| Orange, sweet, ext. | 8028-48-6 | 0.05 |
| | | |

| **Composition 14: Cola Ice** | | |
|---|---|---|
| **Component** | **CAS No.** | **Content [wt.%]** |
| Cellulose (pure) | 9004-34-6 | 67 |
| Aqua | 7732-18-5 | 11.8 |
| N,2,3-Trimethyl-2-isopropylbutamide | 51115-67-4 | 3 |
| 1,2-Propanediol | 57-55-6 | 2.5 |
| Sucrose | 57-50-1 | 1.2 |
| (S)-2-Methyl-5-(1-methyl-2-pyrrolidinyl)pyridine | 13270-56-9 | 1 |
| Sodium chloride | 7647-14-5 | 1 |
| Lime (Citrus aurantifolia), ext. | 90063-52-8 | 1 |
| Orange, sweet, ext. | 8028-48-6 | 0.8 |
| Hydroessential Citrus Limon | 84929-31-7 | 0.8 |
| Potassium carbonate | 584-08-7 | 0.5 |
| Ethyl Vanillin | 121-32-4 | 0.4 |
| Vanillin | 121-33-5 | 0.4 |
| Ethyl maltol | 4940-11-8 | 0.4 |
| Pentane-2,3-dione | 600-14-6 | 0.4 |
| Ethyl butyrate | 105-54-4 | 0.04 |
| Anise alcohol | 105-13-5 | 0.04 |
| Cinnamaldehyde | 104-55-2 | 0.01 |
| | | |

| **Composition 15: Candy Tobacco** | | |
|---|---|---|
| **Component** | **CAS No.** | **Content [wt.%]** |
| Cellulose (pure) | 9004-34-6 | 65 |
| Aqua | 7732-18-5 | 10.3 |
| 1,2-Propanediol | 57-55-6 | 2 |
| Ethyl maltol | 4940-11-8 | 1.6 |
| Ethyl Vanillin | 121-32-4 | 1.6 |
| Sucrose | 57-50-1 | 1.2 |
| (S)-2-Methyl-5-(1-methyl-2-pyrrolidinyl)pyridine | 13270-56-9 | 1 |
| N,2,3-Trimethyl-2-isopropylbutamide | 51115-67-4 | 1 |
| Sodium chloride | 7647-14-5 | 1 |
| 2-hydroxy-3-methylcyclopent-2-enone | 80-71-7 | 0.8 |
| Potassium carbonate | 584-08-7 | 0.5 |
| | | |

| **Composition 16: Blueberry Ice** | | |
|---|---|---|
| **Component** | **CAS No.** | **Content [wt.%]** |
| Cellulose (pure) | 9004-34-6 | 67 |
| Aqua | 7732-18-5 | 11 |
| 1,2-Propanediol | 57-55-6 | 2 |
| N,2,3-Trimethyl-2-isopropylbutamide | 51115-67-4 | 2 |
| Mentha arvensis, ext. | 90063-97-1 | 1.3 |
| Sucrose | 57-50-1 | 1.2 |
| (S)-2-Methyl-5-(1-methyl-2-pyrrolidinyl)pyridine | 13270-56-9 | 1 |
| Sodium chloride | 7647-14-5 | 1 |
| Ethyl butyrate | 105-54-4 | 1 |
| L-menthol | 2216-51-5 | 0.68 |
| Potassium carbonate | 584-08-7 | 0.5 |
| Eucalyptus globulus, ext. | 84625-32-1 | 0.28 |
| trans-Menthone | 89-80-5 | 0.2 |
| Isoamyl acetate | 123-92-2 | 0.11 |
| Neryl acetate | 141-12-8 | 0.02 |
| | | |

| **Composition 17: Ruby Berry Ice** | | |
|---|---|---|
| **Component** | **CAS No.** | **Content [wt.%]** |
| Cellulose (pure) | 9004-34-6 | 67 |
| Aqua | 7732-18-5 | 11 |
| 1,2-Propanediol | 57-55-6 | 2 |
| N,2,3-Trimethyl-2-isopropylbutamide | 51115-67-4 | 2 |
| Mentha arvensis, ext. | 90063-97-1 | 1,5 |
| Sucrose | 57-50-1 | 1,2 |
| (S)-2-Methyl-5-(1-methyl-2-pyrrolidinyl)pyridine | 13270-56-9 | 1 |
| Sodium chloride | 7647-14-5 | 1 |
| L-menthol | 2216-51-5 | 0.68 |
| Potassium carbonate | 584-08-7 | 0.5 |
| Ethyl butyrate | 105-54-4 | 0.45 |
| Acetic acid | 64-19-7 | 0.3 |
| 4-(4-hydroxyphenyl)butan-2-one | 5471-51-2 | 0.3 |
| trans-Menthone | 89-80-5 | 0.2 |
| Butyric acid | 107-92-6 | 0.1 |
| Propionic acid | 79-09-4 | 0.06 |
| trans-hex-2-enal | 6728-26-3 | 0.03 |
| Neryl acetate | 141-12-8 | 0.01 |
| | | |

| **Composition 18: Muffin** | | |
|---|---|---|
| **Component** | **CAS No.** | **Content [wt.%]** |
| Cellulose (pure) | 9004-34-6 | 68 |
| Aqua | 7732-18-5 | 12 |
| 1,2-Propanediol | 57-55-6 | 2.5 |
| N,2,3-Trimethyl-2-isopropylbutamide | 51115-67-4 | 2 |
| (S)-2-Methyl-5-(1-methyl-2-pyrrolidinyl)pyridine | 13270-56-9 | 1 |
| Sucrose | 57-50-1 | 1 |
| Sodium chloride | 7647-14-5 | 1 |
| Ethyl maltol | 4940-11-8 | 0.6 |
| Vanillin | 121-33-5 | 0.6 |
| 3-hydroxy-2-methyl-4-pyrone | 118-71-8 | 0.6 |
| 2-(4-methylthiazol-5-yl)ethanol | 137-00-8 | 0.6 |
| Potassium carbonate | 584-08-7 | 0.5 |
| 2,5-Dimethyl-4-hydroxy-3[2H]-furanone | 3658-77-3 | 0.06 |
| Linalyl acetate | 115-95-7 | 0.06 |
| | | |

| **Composition 19: Peach Ice** | | |
|---|---|---|
| **Component** | **CAS No.** | **Content [wt.%]** |
| Cellulose (pure) | 9004-34-6 | 68 |
| Aqua | 7732-18-5 | 11.8 |
| 1,2-Propanediol | 57-55-6 | 2 |
| N,2,3-Trimethyl-2-isopropylbutamide | 51115-67-4 | 2 |
| (S)-2-Methyl-5-(1-methyl-2-pyrrolidinyl)pyridine | 13270-56-9 | 1 |
| Sucrose | 57-50-1 | 1 |
| Sodium chloride | 7647-14-5 | 1 |
| Potassium carbonate | 584-08-7 | 0.5 |
| Mentha arvensis, ext. | 90063-97-1 | 0.3 |
| L-menthol | 2216-51-5 | 0.18 |
| Linalool | 78-70-6 | 0.1 |
| Linalyl acetate | 115-95-7 | 0.1 |
| Eucalyptus globulus oil | 8000-48-4 | 0.05 |
| | | |

| **Composition 20: Double Mint** | | |
|---|---|---|
| **Component** | **CAS No.** | **Content [wt.%]** |
| Cellulose (pure) | 9004-34-6 | 68 |
| Aqua | 7732-18-5 | 12.7 |
| 1,2-Propanediol | 57-55-6 | 3 |
| N,2,3-Trimethyl-2-isopropylbutamide | 51115-67-4 | 3 |
| I-carvone | 6485-40-1 | 3 |
| L-menthol | 2216-51-5 | 1.77 |
| (S)-2-Methyl-5-(1-methyl-2-pyrrolidinyl)pyridine | 13270-56-9 | 1 |
| Sucrose | 57-50-1 | 1 |
| Sodium chloride | 7647-14-5 | 1 |
| Plant oils / Spear mint oil | 8008-79-5 | 1 |
| Mentha arvensis, ext. | 90063-97-1 | 0.9 |
| Potassium carbonate | 584-08-7 | 0.5 |
| Ammonium chloride | 12125-02-9 | 0.3 |
| Eucalyptus globulus, ext. | 84625-32-1 | 0.075 |
| p-mentha-1,4-diene | 99-85-4 | 0.05 |
| | | |

| **Composition 21: Menthol Ice** | | |
|---|---|---|
| **Component** | **CAS No.** | **Content [wt.%]** |
| Cellulose (pure) | 9004-34-6 | 68 |
| Aqua | 7732-18-5 | 13.15 |
| 1,2-Propanediol | 57-55-6 | 6 |
| N,2,3-Trimethyl-2-isopropylbutamide | 51115-67-4 | 5 |
| Sodium chloride | 7647-14-5 | 3 |
| L-menthol | 2216-51-5 | 2.1 |
| (S)-2-Methyl-5-(1-methyl-2-pyrrolidinyl)pyridine | 13270-56-9 | 1 |
| Potassium carbonate | 584-08-7 | 0.45 |
| Ammonium chloride | 12125-02-9 | 0.4 |
| L-menthan-3-one | 14073-97-3 | 0.3 |
| Menthyl acetate | 16409-45-3 | 0.05 |
| Neomenthol | 491-01-0 | 0.05 |
| I-carvone | 6485-40-1 | 0.05 |
| Isomenthone | 491-07-6 | 0.05 |
| Caryophyllene | 87-44-5 | 0.03 |
| (S)-p-mentha-1,8-diene | 5989-54-8 | 0.03 |

### Example 3 - compositions with (S)-2-Methyl-5-(1-methyl-2-pyrrolidinyl)pyridine and with low nicotine content

Nicotine pouch composition (form of the composition: solid):

| **Composition 22: Menthol Ice** | | |
|---|---|---|
| **Component** | **CAS No.** | **Content [wt.%]** |
| Cellulose (pure) | 9004-34-6 | 68 |
| Aqua | 7732-18-5 | 13.15 |
| 1,2-Propanediol | 57-55-6 | 6 |
| N,2,3-Trimethyl-2-isopropylbutamide | 51115-67-4 | 5 |
| Sodium chloride | 7647-14-5 | 3 |
| L-menthol | 2216-51-5 | 2.1 |
| (S)-2-Methyl-5-(1-methyl-2-pyrrolidinyl)pyridine | 13270-56-9 | 1 |
| Potassium carbonate | 584-08-7 | 0.45 |
| Ammonium chloride | 12125-02-9 | 0.4 |
| L-menthan-3-one | 14073-97-3 | 0.3 |
| Menthyl acetate | 16409-45-3 | 0.05 |
| Neomenthol | 491-01-0 | 0.05 |
| I-carvone | 6485-40-1 | 0.05 |
| Isomenthone | 491-07-6 | 0.05 |
| Caryophyllene | 87-44-5 | 0.03 |
| (S)-p-mentha-1,8-diene | 5989-54-8 | 0.03 |
| Nicotine | 54-11-5 | 0.01 |

Composition of e-cigarette liquid (form of the composition: liquid):

| **Composition 23: Unicon Shake** | | |
|---|---|---|
| **Component** | **CAS No.** | **Content [wt.%]** |
| Glycerol | 56-81-5 | 43.22 |
| 1,2-Propanediol | 57-55-6 | 40.45 |
| Isoamyl acetate | 123-92-2 | 6.08 |
| N,2,3-Trimethyl-2-isopropylbutamide | 51115-67-4 | 5.54 |
| (S)-2-Methyl-5-(1-methyl-2-pyrrolidinyl)pyridine | 13270-56-9 | 0.90 |
| Ethyl butyrate | 105-54-4 | 0.65 |
| Butyl acetate | 123-86-4 | 0.54 |
| cis-3-Hexen-1-ol | 928-96-1 | 0.53 |
| Butanoic acid, 2-methyl-, ethyl ester | 7452-79-1 | 0.44 |
| (E)4-methyl-2-(pent-en-1-yl)-1,3-dioxolane | 94089-21-1 | 0.40 |
| Isopentyl isopentanoater | 659-70-1 | 0.39 |
| (Z)4-methyl-2-(pent-en-1-yl)-1,3-dioxolane | 94089-21-1 | 0.30 |
| 1,3-Dioxolane,4-methyl-2-pentyl | 1599-49-1 | 0.29 |
| Isoamyl butyraten | 106-27-4 | 0.28 |
| Nicotine | 54-11-5 | 0.01 |

### Research

A study was conducted on a group of 100 individuals using compositions 1-21, who had previously used products comprising nicotine and its derivatives in their daily life. The composition was taken twice a day for 60 days.

The results showed the following:
99% of respondents showed a desire to replace the nicotine products used so far with products of the same category, comprising (S)-2-Methyl-5-(1-methyl-2-pyrrolidinyl)pyridine.

The participants in the study emphasised that in their opinion, the composition allowed to achieve faster satisfaction of nicotine craving than previously used nicotine products.

They also did not feel the need to return to nicotine use after the test.

1% of the participants reported no differences in the effects produced by the increased nicotine dose in the products taken, compared to those comprising (S)-2-Methyl-5-(1-methyl-2-pyrrolidinyl)pyridine. It is worth noting that these products were deemed more pleasant to use.

### Conclusions

(S)-2-Methyl-5-(1-methyl-2-pyrrolidinyl)pyridine satisfies nicotine craving faster than substances previously used in nicotine products. Ingestion of a lower concentration of this substance has a similar effect to that of a higher concentration of nicotine or nicotine derivatives. The substance is not addictive and has a more pleasurable sensation than the substances hitherto used in products - nicotine and its derivatives.

## Claims

1. (S)-2-Methyl-5-(1-methyl-2-pyrrolidinyl)pyridine, in an amount of 0.1-5% by weight of the composition, for use in a method for the treatment of nicotine dependence.

2. A pharmaceutical composition comprising (S)-2-Methyl-5-(1-methyl-2-pyrrolidinyl)pyridine, in an amount of 0.1-5% by weight of the composition of oral, inhalation and dermal products for humans, for use in a method for the treatment of nicotine dependence.

3. The pharmaceutical composition comprising (S)-2-Methyl-5-(1-methyl-2-pyrrolidinyl)pyridine for use according to claim 2, **characterised in that** the composition is in the form of a liquid or a solid.

4. The pharmaceutical composition for use according to claim 2 or 3, **characterised in that** it is in the form of an oral, inhalation or dermal human product selected from such as e-cigarettes, e-cigarette liquids, chewing tobaccos, oral pouches, chewing gums, candies, lollipops, oral strips, dragees, jelly beans, lozenges, tablets, snuffs, shisha type smoking mixes, toothpastes, inhalation mixes, inhalers or skin patches.

5. The pharmaceutical composition for use according to any one of the preceding claims 2-4, **characterised in that** it comprises excipients selected from such as at least one substance responsible for the flavour, 1,2-propanediol, N,2,3-trimethyl-2-isopropylbutamide, cellulose, sodium chloride, glycerol and the like.

6. The pharmaceutical composition for use according to any one of the preceding claims 2-5, **characterised in that** it additionally comprises nicotine or its derivatives, in an amount of 0.01-5% by weight of the composition.

7. Use of a composition comprising (S)-2-Methyl-5-(1-methyl-2-pyrrolidinyl)pyridine in an amount of 0.1-5 % by weight as a substitute for nicotine composition in food, inhalation and dermal products for humans.

8. Use according to claim 7, **characterised in that** the composition is in the form of a liquid or a solid.

9. Use according to claim 7 or 8, **characterised in that** the composition is in the form of an oral, inhalation or dermal human product selected from such as e-cigarettes, e-cigarette liquids, chewing tobaccos, oral pouches, chewing gums, candies, lollipops, oral strips, dragees, jelly beans, lozenges, tablets, snuffs, shisha type smoking mixes, toothpastes, inhalation mixes, inhalers or skin patches.

10. Use according to any one of the preceding claims 7-9, **characterised in that** the composition comprises excipients selected from such as at least one substance responsible for the flavour, 1,2-propanediol, N,2,3-trimethyl-2-isopropylbutamide, cellulose, sodium chloride, glycerol and the like.

11. Use according to any one of the preceding claims 7-10, **characterised in that** the composition additionally comprises nicotine or its derivatives in an amount of 0.01-5% by weight of the composition.

12. Use of (S)-2-Methyl-5-(1-methyl-2-pyrrolidinyl)pyridine in an amount of 0.1-5% by weight, as a substitute for nicotine or its derivatives in food, inhalation and dermal products for humans.

13. A composition comprising (S)-2-Methyl-5-(1-methyl-2-pyrrolidinyl)pyridine in an amount of 0.1-5% by weight and excipients selected from such as at least one substance responsible for the flavour, 1,2-propanediol, N,2,3-trimethyl-2-isopropylbutamide, cellulose, sodium chloride, glycerol and the like.

14. The composition according to claim 13, **characterised in that** the composition is in the form of a liquid or a solid in products selected from such as e-cigarettes, e-cigarette liquids, chewing tobaccos, oral pouches, chewing gums, candies, lollipops, oral strips, dragees, jelly beans, lozenges, tablets, snuffs, shisha type smoking mixes, toothpastes, inhalation mixes, inhalers or skin patches.

15. The composition according to claim 13 or 14, **characterised in that** it additionally comprises nicotine or its derivatives in an amount of 0.01-5% by weight.
